# FASCICULE DE BREVET EUROPEEN

(11) **EP 3 307 377 B1**
(45) Date de publication et mention de la délivrance du brevet: **10.07.2024**
(21) Numéro de dépôt: 17733490.1
(22) Date de dépôt: 17.05.2017
(51) Int. Cl.: A61N 1/05, A61N 1/36

(54) **ÉLECTRODE QUADRIPOLAIRE IMPLANTABLE DE STIMULATION DU NERF PHRENIQUE**
IMPLANTIERBARE VIERPOLIGE ELEKTRODE ZUR STIMULATION DES ZWERCHFELLNERVS
IMPLANTABLE QUADRIPOLAR ELECTRODE FOR STIMULATION OF THE PHRENIC NERVE

(30) Priorité: 25.05.2016 FR 1670261
(43) Date de publication de la demande: 18.04.2018
(73) Titulaire: Atrotech (Oy), 33720 Tampere (FI)
(72) Inventeur: RENAUX, SERGE, 34600 Les Aires (FR)
(74) Mandataire: Cassiopi
(86) Numéro de dépôt international: PCT/FR2017/000099
(87) Numéro de publication internationale: WO 2017/203110

(56) Documents cités:
- US-A- 4 940 065
- US-A- 5 919 220
- US-A1- 2003 040 785
- NN: "Atrotech Atrostim Phrenic Nerve Stimulator", 1 April 2012 (2012-04-01), XP055333405, Retrieved from the Internet <URL:http://www.boergel-gmbh.de/fileadmin/Bedienungsanleitungen/Atrostim_PNS.pdf> [retrieved on 20170109]

## Description

### DOMAINE DE L'INVENTION

La présente invention concerne le domaine des électrodes multipolalres, implantables, de neurostimulation séquentielle du nerf phrénique afin de maintenir une respiration artificielle et plus particulièrement de celles, quadripolaires, constituées de deux branches reliées par un pont destiné à les maintenir à une distance bien déterminée l'une de l'autre dans le sens longitudinal du nerf et pourvues, chacune, de deux contacteurs disposés symétriquement autour de l'axe dudit nerf et destinés à être être mis au contact de celui-ci et à être alimentés, en courant électrique de neurostimulation séquentielle, au moyen d'un faisceau de câbles.

Divulgué est également un procédé de fonctionnement et un procédé de pose.

### EXPOSE DE L'ARRIERE PLAN TECHNOLOGIQUE

Le brevet US4940065 décrit une électrode adaptée pour être implantée chirurgicalement autour d'un faisceau nerveux pour assurer sa stimulation sélective agissant sur certaines parties du nerf concerné.

L'électrode comprend un support biocompatible et diélectrique pouvant être formé d'une position ouverte et aplatie jusqu'à une position fermée, encerclant totalement le faisceau du nerf, provocant une contrainte à l'origine de problèmes inflammatoires post opératoires.

Dans sa position ouverte, le support présente une partie de corps principal s'étendant dans la direction d'un axe d'enroulement longitudinal du support et des parties de volet s'étendant transversalement vers l'extérieur à partir des extrémités opposées de la partie de corps principal et de l'axe. Au moins un contact d'électrode est fixé sur une surface intérieure du support et est soudé à un fil conducteur, adapté pour la connexion à un récepteur implanté sur un patient. Dans la réalisation des étapes de procédé pour fabriquer l'électrode, le support constitue à l'origine un tube qui est convenablement coupé pour former le corps principal et ses parties de volet. Le contact de l'électrode est alors fixé à une surface Intérieure du support et soudé au fil conducteur. Elle ne comporte pas de dispositif de fixation autrement dit l'électrode peut coulisser induisant la modification des paramètres stimulation.

La société ATROTECH a conçu, sous la marque « Atrostim », une électrode qui comporte deux paires de contacteurs qui sont disposés symétriquement autour du nerf à une certaine distance l'une de l'autre dans le sens longitudinal de celui-ci.

Durant une séquence de stimulation, qui consiste en une combinaison de quatre courants électriques, les deux pôles opposés activés font office de cathode et d'anode et réciproquement. Il en résulte quatre combinaisons de stimulations engendrant des courants électriques de faible valeur.

Cette technologie a été développée pour réduire la fatigue du nerf sollicité car seules certaines fibres de celui-ci sont activées permettant aux autres de récupérer dans l'intervalle.

L'électrode en question comprend une 1^{ère} branche qui passe sous le nerf et une 2^{ème} qui passe dessus celui-ci. Celle qui passe sous le nerf ressort au travers d'une fenêtre créée chirurgicalement sous celui-ci, la matrice est maintenue en place à l'aide de sutures assurant et le contact avec la nerf. Par contre, celle qui passe sur le nerf n'est pas maintenue par celui-ci et le contact des contacteurs avec le nerf n'est plus assuré. Il est donc nécessaire de le faire par fil de suture.

En outre, l'électrode en question est placée sur chaque nerf phrénique (droite et gauche) selon un acte chirurgical conventionnel invasif par nature (thoracotomie).

Elle ne permet donc pas l'utilisation de trocart (limité à 10mm de diamètre) et donc de la technique thoracoscopique sous vidéo-assistance qui est beaucoup moins invasive.

### RESUME DE L'INVENTION

l'invention est définie par l'électrode selon la revendication 1. Tous les procédés divulgués sont simplement exemplaires et ne font pas partie de la présente invention.

L'invention vise à réaliser une électrode du genre en question implantable par thoracoscopie, à savoir sous vidéo-assistance, au moyen d'un trocart standard, dans le but de réduire le temps d'intervention et les conséquences postopératoires lourdes pour le patient.

Selon l'invention, l'électrode est quadripolaire. En outre, le contact avec le nerf phrénique des deux paires de contacteurs est assuré de par la forme même des branches, le positionnement desdits contacteurs sur lesdites branches et la nature souple et flexible de l'électrode.

L'électrode quadripolaire, implantable, de neurostimulatlon séquentielle du nerf phrénique, se caractérise en ce qu'elle est constituée d'une matrice pourvue de deux branches constituées, chacune, d'un crochet conçu pour venir, sans l'encercler totalement, au contact du nerf phrénique et d'une prolongation, ou languette, conçue pour traverser l'incision correspondante aménagée dans la plèvre et à fixer cette dernière sur le péricarde ;
en ce que chaque crochet possède une ouverture apte à laisser passer le nerf phrénique ;
en ce que lesdits crochets sont reliés par un pont destiné à les maintenir à une distance bien déterminée l'un de l'autre dans le sens longitudinal dudit nerf phrénique et à permettre, de par son élasticité, la rotation des branches l'une par rapport à l'autre en vue du positionnement des crochets autour du nerf phrénique via leurs ouvertures respectives ;
et en ce que lesdits deux crochets sont pourvus, chacun, de deux contacteurs et disposés symétriquement autour de l'axe dudit nerf phrénique, au contact de celui, et conçus pour être alimentés, en courant électrique de neurostimulation séquentielle, au moyen d'un faisceau de quatre câbles.

Selon des caractéristiques additionnelles de l'électrode selon l'invention :
- chaque prolongation, ou languette, comporte un orifice apte à faciliter la fixation de chaque crochet au contact du nerf phrénique sur le péricarde et l'utilisation de clips de chirurgie ;
- la matrice est réalisée en polytétrafluoroéthylène ;
- la matrice est recouverte, sur sa face qui n'est pas en contact avec le nerf, d'une colle silicone apte à fixer la forme de l'électrode tout en lui maintenant la souplesse et la flexibilité optimum pour permettre sa mise en place et son utilisation.

Contrairement au brevet US4940065 qui décrit une électrode :
- adaptée pour être implantée chirurgicalement autour d'un faisceau nerveux pour assurer sa stimulation sélective agissant sur certaines parties du nerf concerné alors que l'électrode selon l'invention est séquentielle et agit sur l'intégralité du nerf ;
- encerclant totalement le faisceau du nerf provocant une contrainte à l'origine de problèmes inflammatoires post opératoires ce qui n'est pas le cas de l'électrode selon l'invention ;
- ne comportant pas de dispositif de fixation autrement dit l'électrode peut coulisser induisant la modification des paramètres stimulation ; ce qui n'est pas le cas de l'électrode selon l'invention ;

Pour au moins une des caractéristiques susmentionnées, l'électrode selon le brevet US4940065 n'est pas applicable au cas présent relatif au nerf phrénique.

Nous sommes en présence de 2 dispositifs non transposables.

En effet, les buts poursuivis, les solutions techniques mises en oeuvre et les résultats obtenus sont totalement différents.

### PRESENTATION DES FIGURES

Les caractéristiques et les avantages de l'invention vont apparaître plus clairement à la lecture de la description détaillée qui suit d'au moins un mode de réalisation préféré de celle-ci donné à titre d'exemple non limitatif et représenté aux dessins annexés.

Sur ces dessins :

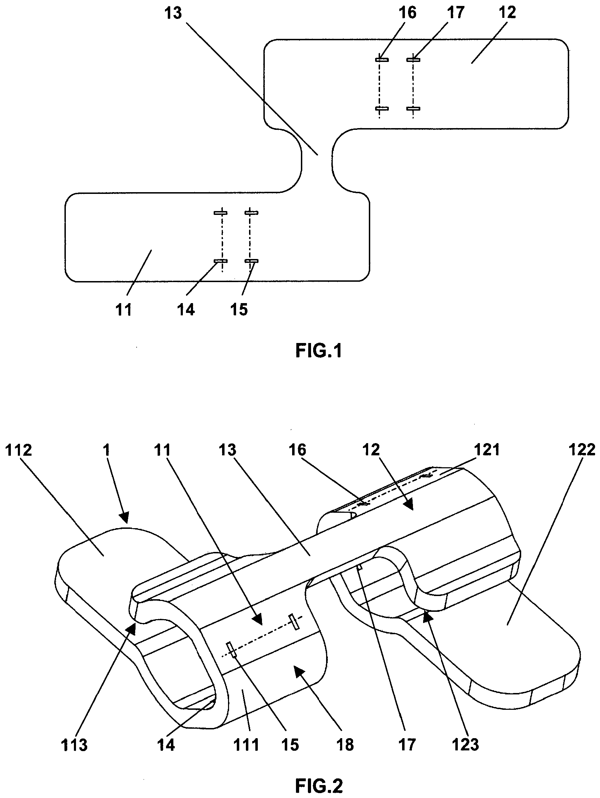

- la figure 1 représente, en vue développée, la matrice de l'électrode mettant en évidence les orifices de maintien des contacteurs ;
- la figure 2 représente, en 3D, la matrice de l'électrode, selon la figure 1, conformée ;

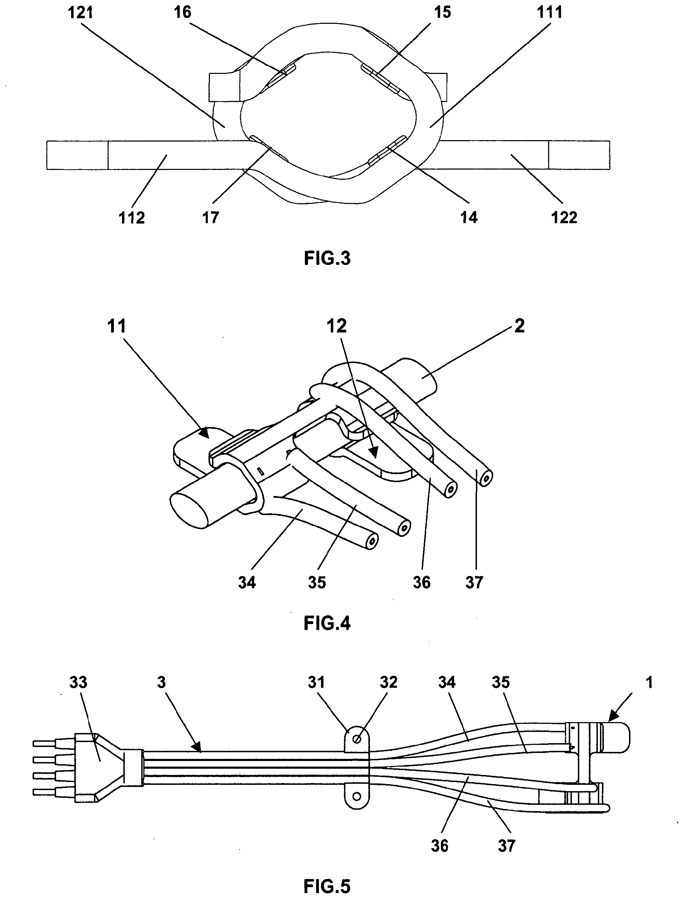

- la figure 3 représente, en vue de profil, l'électrode avec mise en évidence la position des contacteurs ;
- la figure 4 représente, en 3D, l'électrode avec mise en évidence de la position du nerf phrénique et des câbles d'alimentation des contacteurs ;
- la figure 5 représente, en élévation, l'ensemble « électrode et faisceau de câbles ».

### DESCRIPTION DETAILLEE DE L'INVENTION

L'invention concerne une électrode quadripolaire (1), Implantable, de neurostimulation séquentielle du nerf phrénique (2)

Selon les caractéristiques basiques de l'invention telle que représentée aux dessins annexés :
- l'électrode est constituée d'une matrice (1) pourvue de deux branches (11) et (12) constituées, chacune, d'un crochet (111,121) conçu pour venir, sans l'encercler totalement, au contact du nerf phrénique et d'une prolongation (112,122), ou languette, conçue pour traverser l'incision correspondante aménagée dans la plèvre et à fixer cette dernière sur le péricarde ;
- chaque crochet (111,121) possède une ouverture (113,123) apte à laisser passer le nerf phrénique ;
- les crochets (111) et (121) sont reliés par un pont (13) destiné à les maintenir à une distance bien déterminée l'un de l'autre dans le sens longitudinal dudit nerf phrénique (2) et à permettre, de par son élasticité, la rotation des branches (11) et (12) l'une par rapport à l'autre en vue du positionnement des crochets (111) et (121) autour du nerf phrénique via leurs ouvertures respectives (113,123) ;
- lesdits crochets (111) et (121) sont pourvus, chacun, de deux contacteurs (14,15) et (16,17) disposés symétriquement autour de l'axe dudit nerf phrénique (2), au contact de celui, et conçus pour être alimentés, en courant électrique de neurostimulation multi-séquentielle, au moyen d'un faisceau (3) de quatre câbles (34,35,36,37).

Selon des caractéristiques additionnelles de l'invention ;
- chaque prolongation (112,122), ou languette, comporte un orifice (non représenté) apte à faciliter la fixation de chaque crochet au contact du nerf phrénique sur le péricarde et l'utilisation de clips de chirurgie ;
- la matrice (1) est réalisée en polytétrafluoroéthylène ;
- les contacteurs (14,15,16,17), qui sont réalisés en un matériau conducteur électrique, sont insérés dans les parois de la matrice (1) au cours de sa fabrication.
- la matrice (1) est recouverte, sur sa face (18) qui n'est pas en contact avec le nerf (2), d'une colle silicone apte à fixer la forme de l'électrode tout en lui maintenant la souplesse et la flexibilité pour permettre sa mise en place et son utilisation ;
- les câbles électriques (34,35,36,37) sont maintenus entre eux par un dispositif (31) comportant deux orifices (32) conçus pour permettre leur fixation à la plèvre, par fil de suture, afin d'éviter tout risque de tension sur le nerf phrénique (2).

Un procédé de fonctionnement de l'électrode telle que décrite ci-avant consiste à alimenter les contacteurs (14,15,16,17), pris deux à deux, de manière séquentielle, en quatre phases, chaque contacteur devenant à son tour anode ou cathode.

L'électrode (1) et les câbles de liaison (3) selon l'invention sont compatibles avec le stimulateur implanté.

Les extrémités des câbles (34,35,36,37), opposées aux extrémités reliées aux contacteurs (14,15,16,17), sont reliées à une fiche (33) destinée à être connectée au dispositif d'alimentation de l'électrode (1).

Bien entendu, l'homme de métier sera apte à réaliser l'invention telle que décrite et représentée en appliquant et en adaptant des moyens connus sans qu'il soit nécessaire de les décrire ou de les représenter.

Il pourra également prévoir d'autres variantes sans pour cela sortir du cadre de l'invention tel que déterminé par la teneur des revendications.

## Revendications

1. Electrode quadripolaire (1), implantable, de neurostimulation séquentielle du nerf phrénique (2), étant constituée d'une matrice (1) pourvue de deux branches (11) et (12) constituées, chacune, d'un crochet (111,121) conçu pour venir, sans l'encercler totalement, au contact du nerf phrénique et d'une prolongation (112,122), ou languette, conçue pour traverser l'incision correspondante aménagée dans la plèvre et à fixer cette dernière sur le péricarde ;
dans laquelle chaque crochet (111,121) possède une ouverture (113,123) apte à laisser passer le nerf phrénique ;
dans laquelle les crochets (111) et (121) sont reliés par un pont (13) destiné à les maintenir à une distance bien déterminée l'un de l'autre dans le sens longitudinal dudit nerf phrénique (2) et à permettre, de par son élasticité, la rotation des branches (11) et (12) l'une par rapport à l'autre en vue du positionnement des crochets (111) et (121) autour du nerf phrénique via leurs ouvertures respectives (113,123) ;
et dans laquelle lesdits crochets (111) et (121) sont pourvus, chacun, de deux contacteurs (14,15) et (16,17) disposés symétriquement autour de l'axe dudit nerf phrénique (2), au contact de celui, et conçus pour être alimentés, en courant électrique de neurostimulation séquentielle, au moyen d'un faisceau (3) de quatre câbles (34,35,36,37).

2. Electrode, selon la revendication 1, dans laquelle chaque prolongation (112,122), ou languette, comporte un orifice (non représenté) apte à faciliter la fixation de chaque crochet au contact du nerf phrénique sur le péricarde et l'utilisation de clips de chirurgie.

3. Electrode, selon l'une des revendications 1 ou 2, dans laquelle la matrice (1) est réalisée en polytétrafluoroéthylène.

4. Electrode, selon l'une des revendications 1 à 3, dans laquelle les contacteurs (14,15,16,17), qui sont réalisés en un matériau conducteur électrique, sont insérés dans les parois de la matrice (1) au cours de sa fabrication.

5. Electrode, selon l'une des revendications 1 à 4, dans laquelle la matrice (1) est recouverte, sur sa face (18) qui n'est pas en contact avec le nerf (2), d'une colle silicone apte à fixer la forme de l'électrode tout en lui maintenant la souplesse et la flexibilité pour permettre sa mise en place et son utilisation.

6. Electrode, selon l'une des revendications 1 à 5, dans laquelle les câbles électriques (34,35,36,37) sont maintenus entre eux par un dispositif (31) comportant deux orifices (32) conçus pour permettre leur fixation à la plèvre, par fil de suture, afin d'éviter tout risque de tension sur le nerf phrénique (2).

## Patentansprüche

1. Implantierbare, vierpolige Elektrode (1) zur sequenziellen Neurostimulation des Nervus phrenicus (2), bestehend aus einer Matrix (1) mit zwei Schenkeln (11) und (12), die jeweils aus einem Haken (111, 121) bestehen, der so konzipiert ist, dass er, ohne ihn vollständig zu umschließen, mit dem Nervus phrenicus in Kontakt kommt und einer Verlängerung (112, 122) oder Lasche, die so konzipiert ist, dass sie die entsprechende, in der Pleura eingerichtete Inzision durchquert und Letztere am Perikard fixiert;
wobei jeder Haken (111, 121) eine Öffnung (113, 123) aufweist, die geeignet ist, den Nervus phrenicus hindurchzulassen;
wobei die Haken (111) und (121) durch eine Brücke (13) verbunden sind, die dazu bestimmt ist, sie in einem bestimmten Abstand in Längsrichtung des Nervus phrenicus (2) zueinander zu halten und aufgrund ihrer Elastizität die Drehung der Schenkel (11) und (12) zueinander zwecks der Positionierung der Haken (111) und (121) um den Nervus phrenicus über ihre jeweiligen Öffnungen (113, 123) zu ermöglichen;
und wobei die Haken (111) und (121) jeweils mit zwei Kontakten (14, 15) und (16, 17) versehen sind, die symmetrisch um die Achse des Nervus phrenicus (2) im Kontakt mit diesem angeordnet und so ausgelegt sind, dass sie mittels eines Bündels (3) von vier Kabeln (34, 35, 36, 37) mit elektrischem Strom zur sequenziellen Neurostimulation versorgt werden.

2. Elektrode nach Anspruch 1, wobei jede Verlängerung (112, 122) oder Lasche ein Loch (nicht dargestellt) aufweist, das geeignet ist, die Fixierung jedes Hakens im Kontakt mit dem Nervus phrenicus am Perikard und die Verwendung von chirurgischen Clips zu erleichtern.

3. Elektrode nach einem der Ansprüche 1 oder 2, wobei die Matrix (1) aus Polytetrafluorethylen hergestellt ist.

4. Elektrode nach einem der Ansprüche 1 bis 3, wobei die Kontakte (14, 15, 16, 17), die aus einem elektrisch leitfähigen Material hergestellt sind, während ihrer Herstellung in die Wände der Matrix (1) eingesetzt werden.

5. Elektrode nach einem der Ansprüche 1 bis 4, wobei die Matrix (1) auf ihrer Seite (18), die nicht mit dem Nerv (2) im Kontakt ist, mit einem Silikonkleber bedeckt ist, der geeignet ist, die Form der Elektrode zu fixieren und gleichzeitig ihre Flexibilität und Beweglichkeit beizubehalten, um ihre Platzierung und Verwendung zu ermöglichen.

6. Elektrode nach einem der Ansprüche 1 bis 5, wobei die elektrischen Kabel (34, 35, 36, 37) durch eine Vorrichtung (31) mit zwei Öffnungen (32) zusammengehalten werden, die so ausgelegt sind, dass sie mit einem Nahtfaden an der Pleura befestigt werden können, um jedes Risiko einer Spannung auf den Nervus phrenicus (2) zu vermeiden.

## Claims

1. Implantable quadripolar electrode (1) for sequential neurostimulation of the phrenic nerve (2), being constituted of a matrix (1) provided with two branches (11) and (12) each consisting of a hook (111,121) designed to come in contact with the phrenic nerve without totally encircling it, and an extension (112, 122), or strip, designed to cross the corresponding incision arranged in the pleura and fix the latter on the pericardium;
wherein each hook (111, 121) has an opening (113, 123) allowing the phrenic nerve to pass; wherein the hooks (111, 121) are connected by a bridge (13) designed to keep them at a fixed distance from each other in the longitudinal direction of said phrenic nerve (2) and to allow, through its elasticity, the branches (11) and (12) to rotate relative to one another for positioning the hooks (111) and (121) around the phrenic nerve via their respective openings (113, 123); and wherein said hooks (111) and (121) are each provided with two contactors (14, 15) and (16, 17) arranged symmetrically around the axis of said phrenic nerve (2), in contact with it, and designed to be supplied with sequential neurostimulation electrical current by means of a bundle (3) of four cables (34, 35, 36, 37).

2. Electrode, according to claim 1, wherein each extension (112, 122), or strip, comprises an aperture (not shown) that can make it easier to fix each hook in contact with the phrenic nerve on the pericardium and to use surgical clips.

3. Electrode, according to one of claims 1 or 2, wherein the matrix (1) is made of polytetrafluoroethylene.

4. Electrode, according to one of claims 1 to 3, wherein the contactors (14, 15, 16, 17), which are made of an electrically conductive material, are inserted into the walls of the matrix (1) during its manufacture.

5. Electrode, according to one of claims 1 to 4, wherein the matrix (1) is covered, on its surface (18) that is not in contact with the nerve (2), with a silicone adhesive able to fix the shape of the electrode while retaining its elasticity and flexibility so as to enable its installation and use.

6. Electrode, according to one of claims 1 to 5, wherein the electrical cables (34, 35, 36, 37) are held together by a device (31) comprising two apertures (32) designed to enable them to be secured to the pleura, by suture thread, in order to avoid any risk of tension on the phrenic nerve (2).
